# EUROPÄISCHE PATENTSCHRIFT

(11) **EP 1 127 866 B1**
(45) Veröffentlichungstag und Bekanntmachung des Hinweises auf die Patenterteilung: **14.05.2003**
(21) Anmeldenummer: 01102812.3
(22) Anmeldetag: 12.02.2001
(51) Int. Cl.: C07C 67/08

(54) **Verfahren zur Veresterung ungesättigter Cabonsäuren mit ungesättigten Alkoholen unter Vermeidung von Schwarzfärbung und Niederschlagsbildung**
Process for the esterification of unsaturated carboxylic acids from unsaturated alcohols avoiding blackening and precipitation
Procédé d 'estérification d'acides carboxyliques insaturés à partir d'alcools insaturés qui permet d'éviter noircissement et précipitation

(30) Priorität: 24.02.2000 DE 10008509
(43) Veröffentlichungstag der Anmeldung: 29.08.2001
(73) Patentinhaber: Rhein Chemie Rheinau GmbH, 68219 Mannheim (DE)
(72) Erfinder: Herpich, Rüdiger, 68163 Mannheim (DE); Kray, Bernd, 67346 Speyer (DE); Parg, Roland, 51373 Leverkusen (DE); Schmidt, Erich, 68642 Bürstadt (DE); Schöpfer, Stefan, 68766 Hockenheim (DE); Degen, Iris, 68782 Brühl (DE)
(74) Vertreter: Bailly, Peter

(56) Entgegenhaltungen:
- DE-A- 1 493 004
- DE-A- 2 812 978
- DE-A- 2 913 218
- US-A- 2 622 071
- PATENT ABSTRACTS OF JAPAN vol. 1999, no. 05, 31. Mai 1999 (1999-05-31) & JP 11 029524 A (DAICEL CHEM IND LTD), 2. Februar 1999 (1999-02-02)
- HSING-JANG LIU ET AL.: "Convenient procedures for esterification of carboxylic acids" TETRAHEDRON LETTERS., Bd. 46, 1978, Seiten 4461-4464, XP002166834 OXFORD GB
- FRIEDRICH HOFFMANN ET AL.: "Esterification of carboxylic acids by dialkyl phosphonates" JOURNAL OF THE AMERICAN CHEMICAL SOCIETY., Bd. 79, Nr. 17, 11. September 1957 (1957-09-11), Seiten 4759-4761, XP002166835 DC US

## Beschreibung

Die Erfindung betrifft ein Verfahren zur Veresterung ungesättigter Carbonsäuren mit ungesättigten Alkoholen unter Vermeidung von Verfärbung (Schwarzfärbung) und Niederschlagsbildung.

Die Veresterung von Carbonsäuren mit Alkoholen ist bekannt. Im allgemeinen erfolgt diese Reaktion durch Mischen der Säure- und Alkoholkomponente in Gegenwart eines sauren Katalysators und bei höherer Temperatur.

In der Literatur Autorenkollektiv: "Organikum", 16. Auflage, VEB Deutscher Verlag der Wissenschaften, Berlin 1986, S. 402, H. Henecka: "Houben-Weyl, Methoden der organischen Chemie", Band 8, Georg Thieme Verlag Stuttgart 1952, S. 516-526, H. Pielartzik, B. Irmisch-Pielartzik, T. Eicher: "Houben-Weyl, Methoden der organischen Chemie", Band E5/Teil 1, Georg Thieme Verlag Stuttgart 1985, S. 660-684 und W. Riemenschneider: "Ullmann's Enc. Of. Ind. Chemistry" Vol. A9, S. 565-585 werden hierzu starke Säuren, wie Schwefelsäure, Halogenwasserstoffsäuren und Sulfonsäuren, aufgeführt. Nachteilig bei der Verwendung dieser Katalysatoren bei der Veresterung ungesättigter Carbonsäuren mit ungesättigten Alkoholen ist die Schwarz/Braunfärbung und gegebenenfalls Niederschlagsbildung während der Reaktion, sowie Korrosionserscheinungen an Reaktionsbehältern aus Metall. Nachteilig bei der Verwendung dieser Katalysatoren ist weiterhin, dass diese nach der Reaktion durch Auswaschen oder Neutralisieren entfernt bzw. desaktiviert werden müssen. Dies ist mit einem höheren apparativen Aufwand bzw. zusätzlichen Trennverfahren verbunden.

Diese Nachteile sind auch bei der Verwendung von LEWIS-Säuren, wie Bortrifluorid, als Katalysatoren gegeben.

Aus Tetrahedron Letters, **46,** 1978, 4461-4464 ist die Veresterung von CH₂=CH(CH₂)₈COOH mit Propenol in Gegenwart von (CH₃)₂NPOCl₂ bekannt.

DE-A 2913 218 beschreibt eine Veresterungsreaktion unter Verwendung von partiell veresterten Phosphorsäuren, jedoch nicht unter Verwendung ungesättigter Alkohole sowie Carbonsäuren. Auch ein Verfahen zur Mengenreduktion der o.g. Säuren kann entsprechend der o.g. Literatur nach der Methode der azeotropen Veresterung durchgeführt werden. Hierzu wird dem Reaktionsgemisch bei reduzierter Katalysatormenge ein Lösungsmittel hinzugefügt, das in der Lage ist mit Wasser ein Azeotrop zu bilden. Das Reaktionswasser wird dadurch entfernt und der Reaktionsablauf zusätzlich beschleunigt. Nachteilig bei diesem Verfahren ist der Einsatz von Lösungsmitteln, die nach der Veresterung wieder entfernt und gegebenenfalls entsorgt werden müssen.

Eine Veresterung kann entsprechend der o.g. Literatur auch unter Verwendung von sauren Ionenaustauschern durchgeführt werden. Übertragen auf die Veresterung von Gemischen ungesättigter Carbonsäuren mit ungesättigten Alkoholen ist eine Schwarzfärbung der Reaktionsmischung allerdings nicht zu vermeiden. Nachteilig bei diesem Verfahren ist weiterhin der Einbezug des zusätzlichen Verfahrensschrittes der Filtration bzw. Zentrifugation bei nicht kontinuierlichen Herstellprozessen.

Aus der JP-A 11029524 und der DE-A 1493004 sind Veresterungen von nicht ungesättigten Alkoholen unter Verwendung von veresterten Phosphorsäuren bekannt.

Ein Verfahren zur Veresterung von C₁₀-C₂₂-Carbonsäuren mit C₁₀-C₂₂ungesättigten Alkoholen in Gegenwart von sterisch gehinderten Phenolen und partiell veresterten Phosphorsäuren ist diesem Stand der Technik ebensowenig zu entnehmen wie ein Hinweis darauf, dass die Durchführung eines solchen Verfahrens zur Vermeidung von Scharzfärbung und Niederschlagsbildung vorteilhaft sein würde.

Eine weitere Möglichkeit zur Vermeidung der Schwarzfärbung bei Veresterung ist gemäß WO 92/00947 die Verwendung von Additiven mit oxidierender Wirkung, wie Peroxide, Hydroperoxide, Hypochlorite.

In der JP-A 56 070 097 werden hierzu bei der destillativen Reinigung ungesättigter Fettsäuren Additive mit reduzierender Wirkung, wie Hydrazin, Hydroxylamin, Natriumborhydrid, eingesetzt. Gemäß US 4 844 924 erfolgt zur Farbstabilisierung zuerst eine oxidative, danach eine reduktive Bleichung.

Nach der DE-A 3843 938 wird während der Veresterung ungesättigter Carbonsäuren zur Farbaufhellung/-stabilisierung Aktivkohle zugesetzt. Nach DE-A 40 19 788 wird zum gleichen Zweck Aluminiumoxid verwendet.

Nachteilig bei den beschriebenen Methoden mit oxidierenden oder reduzierenden Reagenzien oder Adsorbentien ist nicht nur, dass wie bei der Veresterung mit Ionenaustauschern beschrieben, zusätzlich filtriert oder zentrifugiert werden muss, sondern auch, dass die Reaktionsprodukte nachdunkeln. Weiterhin müssen die zum Teil hochreaktiven und giftigen Zusätze wieder entfernt oder desaktiviert werden, um eine große Bandbreite der Verwendungsmöglichkeit der Ester zuzulassen. Die Verwendung komplexer Aluminium- oder Borhydride kann zur Bildung brennbarer Gase führen.

Aufgabe der Erfindung ist es nun, ein Verfahren zur Veresterung ungesättigter Carbonsäuren mit ungesättigten Alkoholen zur Verfügung zu stellen, mit dem sich hellfarbige und bei Lagerung farbstabile Produkte herstellen lassen. Das Verfahren soll auch anwendbar sein auf Gemische von Carbonsäuren und Alkholen mit nicht identifizierten Begleitstoffen, wie sie als Naturstoffe und in der Oleochemie anfallen.

Es wurde jetzt gefunden, dass durch Verwendung von partiell veresterten Phosphorsäuren zusammen mit sterisch gehinderten Phenolen und Einleiten von Inertgas in die Reaktionsmischung während der Umesterungsreaktion hellfarbige und farbstabile Reaktionsprodukte erhalten werden.

Gegenstand der Erfindung ist daher ein Verfahren zur Veresterung von ungesättigten Carbonsäuren mit ungesättigten Alkoholen, das dadurch gekennzeichnet ist, dass man die Veresterung in Gegenwart von sterisch gehinderten Phenolen und partiell veresterten Phosphorsäuren durchführt.

Als ungesättigte Alkohole kommen einfach oder mehrfach ungesättigte, geradkettige oder verzweigte Monoalkohole mit 10 bis 22 Kohlenstoffatomen, bevorzugt 10 bis 18 Kohlenstoffatomen in Betracht. Zu nennen sind beispielsweise: Decenol, Undecenol, Dodecenol, Tridecenol, Tetradecenol, Pentadecenol, Hexadecenol, Heptadecenol, Octadecenol, Nonadecenol, Eicosenol, Heneicosenol, Docosenol, Decadienol, Dodecadienol, Tetradecadienol, Hexadecadienol, Octadecadienol, Eicosadienol, Docosadienol. Bevorzugt sind: Decenol, Dodecenol, Tetradecenol, Hexadecenol, Octadecenol. Ganz besonders bevorzugt sind: Decenol, Dodecenol, Palmitoleyalkohol, Oleylalkohol.

Als ungesättigte Carbonsäuren können in das erfindungsgemäße Verfahren ein- oder mehrfach ungesättigte, geradkettige oder verzweigte Monocarbonsäuren mit 10 bis 22 Kohlenstoffatomen, bevorzugt 14 bis 18 Kohlenstoffatomen, eingesetzt werden.

Zu nennen sind als ungesättigte Carbonsäuren beispielsweise: Decensäure, Undecensäure, Dodecensäure, Tridecensäure, Tetradecensäure, Pentadecensäure, Hexadecensäure, Heptadecensäure, Octadecensäure, Nonadecensäure, Eicosensäure, Heneicosensäure, Docosensäure, Decadiensäure, Dodecadiensäure, Tetradecadiensäure, Hexadecadiensäure, Octadecadiensäure, Octadecatriensäure, Eicosatetraensäure. Bevorzugt sind: Tetradecensäure, Hexadecensäure, Octadecensäure, Octadecadiensäure sowie Octadecatriensäure. Ganz besonders bevorzugt sind: Myristoleinsäure, Palmitoleinsäure, Ölsäure, Linolsäure sowie Linolensäure.

Sowohl die ungesättigten Alkohole als auch die ungesättigten Carbonsäuren können einzeln als auch im Gemisch untereinander in das erfindungsgemäße Verfahren eingesetzt werden.

Wie oben erwähnt, wird das erfindungsgemäße Verfahren in Gegenwart von partiell veresterten Phosphorsäuren und in Gegenwart von sterisch gehinderten Phenolen durchgeführt.

Als partiell veresterte Phosphorsäure kommen Phosphorsäuremono- und Phosphorsäurediester mit Alkylresten in Betracht, die 1 bis 10, bevorzugt 1 bis 8 Kohlenstoffatome in geradkettiger, verzweigter oder ringförmiger Anordnung aufweisen. Als Beispiele für partiell veresterte Phosphorsäuren, die in das erfindungsgemäße Verfahren eingesetzt werden können, sind zu nennen: Phosphorsäuredioctylester, Phosphorsäuredihexylester, Phosphorsäuredibutylester, Phosphorsäuremonooctylester, Phosphorsäuremonohexylester, Phosphorsäuremonobutylester. Bevorzugt sind: Phosphorsäuredioctylester, Phosphorsäuredihexylester, Phosphorsäuremonooctylester, Phosphorsäuremonohexylester. Ganz besonders bevorzugt sind: Gemische von Phosphorsäuredi(2-ethyl-hexyl)ester, Phosphorsäuremono(2-ethyl-hexyl)ester, Phosphorsäuredi-n-hexylester, Phosphorsäuremono-n-hexylester in beliebigem Mischungsverhältnis.

Als sterisch gehinderte Phenole können in das erfindungsgemäße Verfahren solche Phenole eingesetzt werden, denen einkernige oder mehrkernige, bevorzugt 1 - 4 kernige Phenole mit mindestens zwei Substituenten, bevorzugt 3 Substituenten, zugrundeliegen, von denen zwei Substituenten in ortho-Stellung zu den OH-Gruppen stehen, und die jeweils 1 bis 80, bevorzugt 1 bis 60 Kohlenstoffatome aufweisen und bis zu 18, bevorzugt bis 12 Sauerstoffatome tragen.

Beispiele hierzu sind: 2,6-Di-tert.-butyl-4-methyl-phenol, 2,6-Di-tert.-butyl-4-ethylphenol, 2,6-Di-tert.-butyl-4-nonyl-phenol, 2,6-Di-tert.-butyl-4-sec.-butyl-phenol, 2,6-Di-benzyl-4-methyl-phenol, 3-(3,5-Di-tert.-butyl-4-hydroxyphenyl)propansäuremethylester, 3-(3,5-Di-tert-butyl-4-hydroxyphenyl)propansäureoctadecylester, 2,2'-Methylenbis(6-tert.-butyl-4-methylphenol), 2,2'-Methylenbis(6-tert.-butyl-4-ethylphenol), 2,2'-Methylenbis(4-methyl-6-cyclohexylphenol), 4,4'-Methylenbis(2,6-ditert.-butylphenol), 2,2'-Ethylidenbis(4,6-di-tert.-butylphenol), 4,4'-Butylidenbis(6-tert.-butyl-3-methylphenol), 2,2'-Isobutylidenbis(4,6-dimethylphenol), 1,3,5-Trimethyl-2,4,6-tris(3,5-di-tert.-butyl-4-hydroxybenzyl)benzol, Hexamethylenbis(3,5-di-tert.-butyl-4-hydroxy-hydrocinnamat), Penta-erythrityl-tetrakis[3-(3,5-du-tert.-butyl-4-hydroxyphenyl)-propionat], 1,3,5-Trimethyl-2,4,6-tris(3,5-di-tert.-butyl-4-hydroxybenzyl)benzol. Bevorzugt sind 2,6-Di-tert.-butyl-4-methyl-phenol, 2,2'-Methylenbis(6-tert.-butyl-4-methylphenol), 2,2'-Methylenbis(4-methyl-6-cyclohexylphenol), Pentaerythrityl-tetrakis[3-(3,5-di-tert.-butyl-4-hydroxyphenyl)-propionat] und 1,3,5-Trimethyl-2,4,6-tris(3,5-di-tert.-butyl-4-hydroxybenzyl)benzol. Ganz besonders bevorzugt sind: 2,6-Di-tert.-butyl-4-methyl-phenol, Pentaerythrityl-tetrakis[3-(3,5-ditert.-butyl-4-hydroxyphenyl)-propionat] und 1,3,5-Trimethyl-2,4,6-tris(3,5-di-tert.butyl-4-hydroxybenzyl)benzol.

Sowohl die sterisch gehinderten Phenole als auch die partiell veresterten Phosphorsäuren können alleine oder im Gemisch untereinander eingesetzt werden. Das jeweilige Mischungsverhältnis ist leicht durch entsprechende Vorversuche einzustellen und richtet sind insbesondere nach den eingesetzten Alkoholen und Säuren.

Die partiell veresterten Phosphorsäuren werden im allgemeinen in Mengen von 0,01 bis 5 Gew.-%, bezogen auf die Gesamtmenge an Alkohol und Carbonsäure, eingesetzt. Bevorzugt sind Mengen von 0,1 bis 1,5 Gew.-%. Ganz besonders bevorzugt sind Mengen von 0,3 bis 1,0 Gew.-%.

Die Menge an sterisch gehinderten Phenolen beträgt im allgemeinen 0,01 bis 3 Gew.-%, bezogen auf die Gesamtmenge an Alkohol und Carbonsäuren. Bevorzugt sind Mengen von 0,05 bis 1,0 Gew.-%. Ganz besonders bevorzugt sind Mengen von 0,08 bis 0,5 Gew.-%.

Bei dem erfindungsgemäßen Verfahren beträgt das Verhältnis von partiell veresterten Phosphorsäuren zu sterisch gehinderten Phenolen üblicherweise 50 bis 0,1:1, bevorzugt 10 bis 1:1.

Die ungesättigten Carbonsäuren und die Alkohole werden erfindungsgemäß im Molverhältnis von 1:2 bis 0,5, bevorzugt 1:1,1 bis 0,9 eingesetzt.

In einer bevorzugten Ausführungsform des erfindungsgemäßen Verfahrens wird die Veresterung unter Einleiten von Inertgas, wie Stickstoff oder Argon, durchgeführt. Die Menge an Inertgas ist nicht kritisch und kann leicht durch entsprechende Vorversuche ermittelt werden.

In einer bevorzugten Ausführungsform wird das erfindungsgemäße Verfahren bei Temperaturen im Bereich von 130 bis 200°C durchgeführt, insbesondere bei Temperaturen im Bereich von 150 bis 180°C. Die ungesättigten Monocarbonsäuren und ungesättigten Alkohole werden mit den partiell veresterten Phosphorsäuren und sterisch gehinderten Phenolen ohne Zusatz von Lösungsmitteln oder Schleppmitteln unter Einleiten von Inertgas zur Reaktion gebracht. Das entstehende Reaktionswasser wird mit dem Inertgasstrom abgeführt in üblicher Weise, z.B. durch Nachschalten eines Liebig-Kühlers.

### Beispiele

Die %-Angaben ohne nähere Bezeichnung beziehen sich auf Flächen-% im jeweiligen Gaschromatogramm der Alkohol- bzw. Carbonsäurekomponente.

### Vergleichsbeispiel 1

100,0 g einer Alkoholkomponente, bestehend aus: 75 Gew.-% Oleylalkohol, 25 Gew.-% Hexadecylalkkohol und 105,6g einer Säurekomponente, bestehend aus: 70 % Ölsäure, 8,3 % Linolsäure, 5,3 % Palmitoleinsäure, 16,4 % Säuren mit einer Kettenlänge von C14-C18 werden mit 0,6 g para-Toluolsulfonsäure gemischt und erhitzt bis die Säurezahl unter 10 mg KOH/g sinkt.

Schwarzfärbung des Reaktionsansatzes und Niederschlagsbildung.
Farbzahl der Mischung Hess-Ivess
(Hess-Ivess-Farbzahl: DKG-Methode Nr.: F010.1., der Deutschen Gesellschaft für wissenschaftliche und angewandte Kosmetik)
vor der Reaktion: 8,8 nach der Reaktion: 300,2

### Vergleichsbeispiel 2

70,0 g einer Alkoholkomponente, bestehend aus: 93 % Oleylalkohol, 7 % Alkohole mit einer Kettenlänage von C12-C22 und 75,0 g einer Säurekomponente, bestehend aus: 70 % Ölsäure, 8,3 % Linolsäure, 5,3 % Palmitoleinsäure, 16,4 % Säuren mit einer Kettenlänge von C14-C18 werden mit 2,6 g Ionenaustauscher Deloxan® ASP 1/9 (Fa. Degussa, Propyl(3-sulfonsäure)siloxan/Siliziumdioxid-Copolykondensat) gemischt und erhitzt bis die Säurezahl unter 20 mg KOH/g sinkt.

Dunkelfärbung des Reaktionsansatzes.
Farbzahl der Mischung Hess-Ivess
vor der Reaktion: 8,8 nach der Reaktion: 116,4

### Vergleichsbeispiel 3

70,0 g einer Alkoholkomponente, bestehend aus: 93 % Oleylalkohol, 7 % Alkohole mit einer Kettenlänage von C12-C22 und 75,0g einer Säurekomponente, bestehend aus: 70 % Ölsäure, 8,3 % Linolsäure, 5,3 % Palmitoleinsäure, 16,4 % Säuren mit einer Kettenlänge von C14-C18 werden mit 0,5 g Phosphorsäure gemischt und erhitzt bis die Säurezahl unter 10 mg KOH/g sinkt.

Dunkelfärbung des Reaktionsansatzes, Bildung brauner Flocken.
Farbzahl der Mischung Hess-Ivess
vor der Reaktion: 0,0 nach der Reaktion: 97,5

### Beispiel 1

70,0 g einer Alkoholkomponente, bestehend aus: 93 % Oleylalkohol, 7 % Alkohole mit einer Kettenlänge von C12-C22 und 75,0g einer Säurekomponente, bestehend aus: 70 % Ölsäure, 8,3 % Linolsäure, 5,3 % Palmitoleinsäure, 16,4 % Säuren mit einer Kettenlänge von C14-C18 werden mit 0,73g Phosphorsäuredi(2-ethylhexyl)ester/Phosphorsäuremono-2-ethylhexylester-Gemisch und 0,15 g 2,6-Di-tert.butyl-4-methylphenol gemischt und erhitzt bis die Säurezahl unter 10mg KOH/g sinkt.

Das Estergemisch liegt als klare, blassgelbe Flüssigkeit vor.
Farbzahl der Mischung Hess-Ivess
vor der Reaktion: 8,8 nach der Reaktion (Standzeit 1 Jahr): 14,2

### Beispiel 2

65,8 g einer Alkoholkomponente, bestehend aus: 93 % Oleylalkohol, 7 % Alkohole mit einer Kettenlänage von C12-C22 und 71,1g einer Säurekomponente, bestehend aus: 35 Gew.-% Ölsäure, 55 Gew.-% Linolsäure und 10 Gew.-% Octadecatriensäure werden mit 0,69 g Phosphorsäuredi(2-ethylhexyl)ester/Phosphorsäuremono-2-ethylhexylester-Gemisch und 0,27 g Pentaerythrityltetrakis[3-(3,5-di-tert.-butyl-4-hydroxyphenyl)-propionat] gemischt und erhitzt bis die Säurezahl unter 10mg KOH/g sinkt.

Das Estergemisch liegt als klare, gelbe Flüssigkeit vor.
Farbzahl der Mischung Hess-Ivess
vor der Reaktion: 2,1 nach der Reaktion: 39,7

### Beispiel 3

74,8 g einer Alkoholkomponente, bestehend aus: 93 % Oleylalkohol, 7 % Alkohole mit einer Kettenlänage von C12-C22 und 80 g einer Säurekomponente, bestehend aus: 23 Gew.-% Ölsäure, 10 Gew.-% Palmitinsäure, 53 Gew.-% Linolsäure, 9 Gew.-% Linolensäure werden mit 0,4 g Phosphorsäuredi(2-ethylhexyl)ester/Phosphorsäuremono-2-ethylhexylester-Gemisch und 0,31 g 1,3,5-Trimethyl-2,4,6-tris-(3,5-di-tert.-butyl-4-hydroxybenzyl)benzol gemischt und erhitzt bis die Säurezahl unter 10 mg KOH/g sinkt.

Das Estergemisch liegt als klare, orangefarbene Flüssigkeit vor.
Farbzahl der Mischung Hess-Ivess
vor der Reaktion: 0,2 nach der Reaktion: 61,7

### Beispiel 4

80,0 g einer Alkoholkomponente, bestehend aus: 93 % Oleylalkohol, 7 % Alkohole mit einer Kettenlänage von C12-C22 und 80 g einer Säurekomponente, bestehend aus: 70 % Ölsäure, 8,3 % Linolsäure, 5,3 % Palmitoleinsäure, 16,4 % Säuren mit einer Kettenlänge von C14-C18 werden mit 0,4g Phosphorsäuredi(2-ethylhexyl)-ester/Phosphorsäuremono-2-ethylhexylester-Gemisch und 0,15 Pentaerythrityltetrakis[3-(3,5-di-tert.-butyl-4-hydroxyphenyl)-propionat] gemischt und erhitzt bis die Säurezahl unter 10 mg KOH/g sinkt.

Das Estergemisch liegt als klare, orangefarbene Flüssigkeit vor.
Farbzahl der Mischung Hess-Ivess
vor der Reaktion: 0,0 nach der Reaktion: 61,7

## Patentansprüche

1. Verfahren zur Veresterung von ungesättigten C₁₀-C₂₂-Carbonsäuren mit ungesättigten C₁₀-C₂₂-Alkoholen, **dadurch gekennzeichnet, dass** man die Veresterung in Gegenwart von sterisch gehinderten Phenolen und partiell veresterten Phosphorsäuren durchführt.

2. Verfahren nach Anspruch 1, **dadurch gekennzeichnet, dass** man die Veresterung unter Einleiten von Inertgas durchführt.

3. Verfahren nach Anspruch 1, **dadurch gekennzeichnet, dass** die Veresterung in Gegenwart von 0,1 bis 3 Gew.-% an sterisch gehinderten Phenolen, bezogen auf die Gesamtmenge an Alkohol- und Carbonsäure, durchgeführt wird.

4. Verfahren nach Anspruch 1, **dadurch gekennzeichnet, dass** die Veresterung in Gegenwart von 0,01 bis 5 Gew.-% an partiell veresterten Phosphorsäuren, bezogen auf die Gesamtmenge an Alkohol- und Carbonsäure, durchgeführt wird.

5. Verfahren nach Anspruch 1, **dadurch gekennzeichnet, dass** die ungesättigten Carbonsäuren und die ungesättigten Alkohole im Molverhältnis von 1:2 bis 0,5 eingesetzt werden.

6. Verfahren nach Anspruch 1, **dadurch gekennzeichnet, dass** das Verhältnis von partiell veresterten Phosphorsäuren zu sterisch gehinderten Phenolen 50 bis 0,1:1 beträgt.

## Claims

1. A process for the esterification of unsaturated C₁₀-C₂₂ carboxylic acids with unsaturated C₁₀-C₂₂ alcohols, **characterised in that** esterification is performed in the presence of sterically hindered phenols and partially esterified phosphoric acids.

2. A process according to claim 1, **characterised in that** esterification is performed with introduction of inert gas.

3. A process according to claim 1, **characterised in that** esterification is performed in the presence of 0.1 to 3 wt.% of sterically hindered phenols, relative to the total quantity of alcohol and carboxylic acid.

4. A process according to claim 1, **characterised in that** esterification is performed in the presence of 0.01 to 5 wt.% of partially esterified phosphoric acids, relative to the total quantity of alcohol and carboxylic acid.

5. A process according to claim 1, **characterised in that** the unsaturated carboxylic acids and the unsaturated alcohols are used in a molar ratio of 1:2-0.5.

6. A process according to claim 1, **characterised in that** the ratio of partially esterified phosphoric acids to sterically hindered phenols is 50-0.1:1.

## Revendications

1. Procédé pour l'estérification d'acides carboxyliques insaturés en C₁₀-C₂₂ par des alcools insaturés en C₁₀-C₂₂, **caractérisé en ce que** l'estérification est réalisée en présence de phénols objets d'un empêchement stérique et d'acides phosphoriques partiellement estérifiés.

2. Procédé selon la revendication 1, **caractérisé en ce que** l'estérification est réalisée sous injection d'un gaz inerte.

3. Procédé selon la revendication 1, **caractérisé en ce que** l'estérification est réalisée en présence de 0,1 à 3 % en poids de phénols objets d'un empêchement stérique par rapport à la quantité totale d'alcool et d'acide carboxylique.

4. Procédé selon la revendication 1, **caractérisé en ce que** l'estérification est réalisée en présence de 0,01 à 5 % en poids d'acides phosphoriques partiellement estérifiés par rapport à la quantité totale d'alcool et d'acide carboxylique.

5. Procédé selon la revendication 1, **caractérisé en ce que** les acides carboxyliques insaturés et les alcools insaturés sont mis en oeuvre dans des proportions molaires de 1:2 à 0,5.

6. Procédé selon la revendication 1, **caractérisé en ce que** le rapport entre les acides phosphoriques partiellement estérifiés et les phénols objets d'un empêchement stérique va de 50 à 0,1:1.
